# EUROPÄISCHE PATENTSCHRIFT

(11) **EP 1 433 424 B1**
(45) Veröffentlichungstag und Bekanntmachung des Hinweises auf die Patenterteilung: **07.06.2006**
(21) Anmeldenummer: 03028328.7
(22) Anmeldetag: 10.12.2003
(51) Int. Cl.: A61B 17/16, A61B 17/32, A61F 2/18

(54) **Knorpelstanzvorrichtung**
Cartilage punching device
Dispositif de poinçonnage pour cartilage

(30) Priorität: 27.12.2002 DE 20219994 U
(43) Veröffentlichungstag der Anmeldung: 30.06.2004
(73) Patentinhaber: Heinz Kurz GmbH Medizintechnik, 72144 Dusslingen (DE)
(72) Erfinder: Hüttenbrink, Karl-Bernd, Prof. Dr. med., 01326 Dresden (DE); Steinhardt, Uwe, 72145 Hirrlingen (DE)
(74) Vertreter: Möbus, Daniela

(56) Entgegenhaltungen:
- DE-A- 2 927 160
- US-A- 4 641 651
- US-A- 5 716 405
- US-A- 5 823 971
- US-A- 5 827 316

## Beschreibung

Die Erfindung betrifft eine Knorpelstanzvorrichtung mit der Knorpelplättchen, die in ihrem Zentrum eine Öffnung aufweisen sollen, aus einem Knorpelstück herausgestanzt werden können.

Bei der Totalrekonstruktion der menschlichen Mittelohr-Gehörknöchelchenkette wird eine Prothese verwendet, die an Stelle der Gehörknöchelchen zwischen Trommelfell und der Steigbügelfußplatte im Bereich des ovalen Fensters, das das Mittelohr vom Innenohr trennt, angeordnet wird. Dabei besteht die Gefahr, dass nach der Operation die Prothese kippen oder umfallen kann. Weiter kann es nach der Implantation im Rahmen des Heilungsprozesses zu Narbenzügen kommen, die die Prothese in ihrer Position auf der Fußplatte verändern können. Um dies zu vermeiden, wird an der Stelle, an der die Prothese an der Fußplatte des Steigbügels anliegt, eine Befestigung in Form eines Knorpelplättchens vorgesehen. Der dazu notwenige Knorpel kann beispielsweise aus dem Dragus oder der Concha des äußeren Ohrs entnommen werden. Im Zentrum des Plättchens befindet sich eine Öffnung oder Bohrung, die das Ende beispielsweise in Form eines Stempels der Prothese aufnimmt. Damit ist die Prothese sicher und definiert im Zentrum dieses Knorpelplättchens und damit auf der Steigbügelfußplatte im Bereich des ovalen Fensters befestigt.

Das für die Befestigung der Prothese notwendige Knorpelplättchen ist sehr klein und muss außerdem in seinem Zentrum noch eine Öffnung aufweisen. Es ist ohne eine spezielle Vorrichtung sehr schwierig und nur mit großem handwerklichem Geschick möglich, ein solches Knorpelplättchen herzustellen

Aus US 4641651 ist bekannt ein Knorpelplättchen ohne Öffnung zu verwenden. Die Vorrichtung zur Herstellung eines solchen Knorpelplättchens besteht aus einer Außenstanzvorrichtung mit der der äußere Umfang des Knorpelplättchens ausgestanzt wird und einer Ausstoßvorrichtung mit der das so aus dem Knorpel ausgestanzte Knorpelplättchen aus der Knorpelstanzvorrichtung ausgestoßen werden kann.

Der Erfindung liegt daher die Aufgabe zugrunde, eine Vorrichtung anzugeben, mit der ein solches kleines Knorpelplättchen mit einer Öffnung im Zentrum einfach und schnell hergestellt werden kann.

Die gestellte Aufgabe wird erfindungsgemäß durch die Knorpelstanzvorrichtung, die die im Hauptanspruch aufgeführten Merkmale aufweist, gelöst. Die Unteransprüche geben bevorzugte Weiterbildungen an.

Die erfindungsgemäße Knorpelstanzvorrichtung zur Herstellung eines mit einer Öffnung versehenen Knorpelplättchens besteht aus einer Außenstanzvorrichtung, mit der der äußere Umfang des Knorpelplättchens ausgestanzt wird, einer Öffnungsstanzvorrichtung, mit der die Öffnung im Inneren des Knorpelplättchens ausgestanzt wird, und einer Ausstoßvorrichtung, mit der das so aus dem Knorpel ausgestanzte Knorpelplättchen aus der Knorpelstanzvorrichtung ausgestoßen werden kann.

In einer vorteilhaften Ausführungsform sind die Außenstanzvorrichtung, die Öffnungsstanzvorrichtung und die Ausstoßvorrichtung in einer Linie hintereinander angeordnet.

Es ist weiter vorteilhaft, wenn das Ausstanzen des Umfangs des Knorpelplättchens und das Ausstanzen der Öffnung im Knorpelplättchen in einem Arbeitsgang erledigt werden können, ohne dass die Knorpelstanzvorrichtung am Knorpel nochmals angesetzt werden muss. Dazu ist in einer vorteilhaften Ausführungsform die Öffnungsstanzvorrichtung so ausgebildet, dass sie direkt durch die Außenstanzvorrichtung hindurch die Öffnung ausstanzen kann, während die Außenstanzvorrichtung den Umfang des Knorpelplättchens ausstanzt. Dazu kann die Öffnungsstanzvorrichtung beispielsweise einen innen hohl ausgebildeten Öffnungsstanzvorrichtungsgriff und eine stabförmige Verlängerung, an deren Spitze ein Öffnungsstanzstab, der das eigentliche Stanzen der Öffnung vornimmt, angeordnet ist, aufweisen. Die stabförmige Verlängerung gleitet in der innen hohl ausgebildeten Außenstanzvorrichtung. Somit ist die Öffnungsstanzvorrichtung mit dem Öffnungsstanzvorrichtungsgriff durch die Außenstanzvorrichtung hindurch bedienbar, um die Öffnung im Knorpelplättchen auszustoßen.

In einer weiteren vorteilhaften Ausführungsform ist die Ausstoßvorrichtung ebenfalls so ausgebildet, dass sie durch die Öffnungsstanzvorrichtung hindurch betätigbar ist. Sie weist dazu eine Ausstoßstange, die an einem Ausstoßgriff befestigt ist, auf. Die Ausstoßstange gleitet in der innen hohl ausgebildeten stabförmigen Verlängerung der Öffnungsstanzvorrichtung und drückt, wenn der Ausstoßgriff betätigt wird, diese stabförmige Verlängerung der Öffnungsstanzvorrichtung nach vorne in die Außenstanzvorrichtung hinein, sodass das sich in der Außenstanzvorrichtung befindliche ausgestanzte Knorpelplättchen aus der Außenstanzvorrichtung und damit der gesamten Knorpelstanzvorrichtung ausgestoßen wird.

Damit die Außenstanzvorrichtung auch unabhängig von der Öffnungsstanzvorrichtung betätigt werden kann, wenn beispielsweise ein Knorpelplättchen ausgestanzt werden soll, dass keine Öffnung aufweist, weist die Außenstanzvorrichtung in einer vorteilhaften Ausführungsform einen Außenstanzgriff und einen daran angeordneten und mit dem Außenstanzgriff betätigbaren Stanzfortsatz aus. Der Stanzfortsatz weist eine Öffnung auf, die die Form des ausgestanzten Knorpelplättchens bestimmt. Der Außenstanzgriff muss innen hohl ausgebildet sein, damit die stabförmige Verlängerung der Öffnungsstanzvorrichtung und der daran angeordnete Öffnungsstanzstab durch ihn hindurch geführt werden können.

Es ist weiter vorteilhaft, wenn zwischen der Außenstanzvorrichtung und der Öffnungsstanzvorrichtung eine Feder angeordnet ist, sodass sich nach dem Ineinanderschieben dieser beiden Vorrichtungen beim Stanzen, diese wieder voneinander lösen und somit nach dem Betätigen der Ausstoßvorrichtung, um das gestanzte Knorpelplättchen auszustoßen, wieder für einen neuen Stanzvorgang bereit stehen. Die Feder kann beispielsweise in Form einer Schraubenfeder, die auf der stabförmigen Verlängerung der Öffnungsstanzvorrichtung gleitet, ausgebildet sein.

Ein Ausführungsbeispiel einer erfindungsgemäß ausgebildeten Knorpelstanzvorrichtung wird nachfolgend anhand der beiliegenden Zeichnung erläutert. In den Zeichnungen sind gleiche Elemente in allen Zeichnungsfiguren mit den gleichen Bezugszahlen gekennzeichnet.

Es zeigen:
- Fig. 1: einen Explosionsdarstellung einer Ausführungsform der erfindungsgemäßen Knorpelstanzvorrichtung;
- Fig. 2: die in Fig. 1 dargestellte Knorpelstanzvorrichtung in ihrem zusammengebauten Zustand; und
- Fig. 3: einen schematischen Querschnitt durch das Mittelohr eines Menschen, die zeigt, wie das mit der erfindungsgemäßen Knorpelstanzvorrichtung ausgestanzte Knorpelplättchen für die Verankerung einer Gehörknöchelchenprothese Verwendung findet.

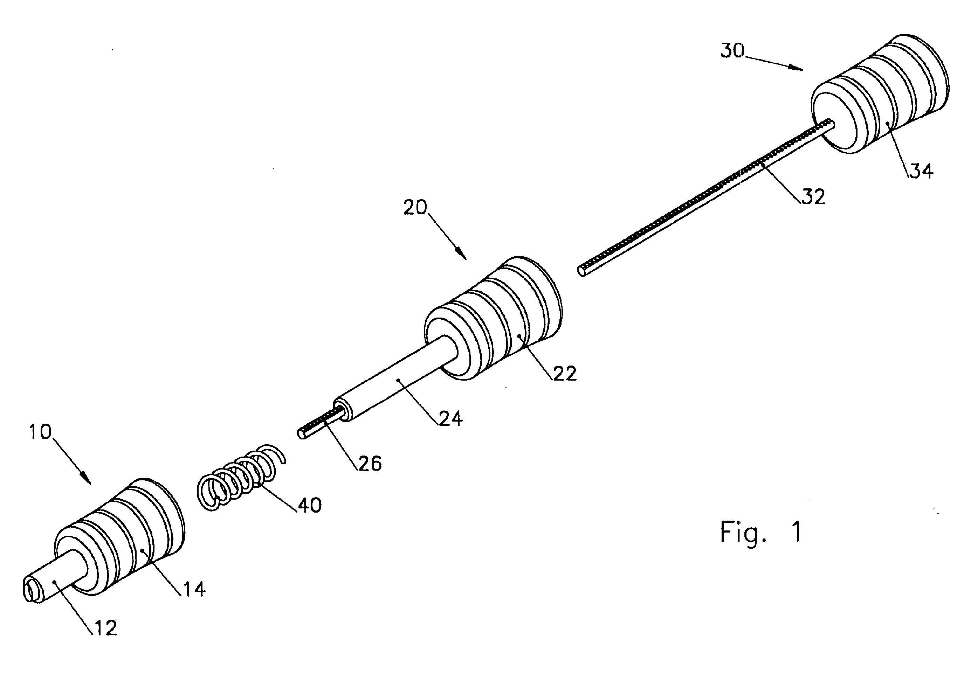

Fig. 1 zeigt eine Explosionsdarstellung einer Ausführungsform der erfindungsgemäßen Knorpelstanzvorrichtung. Die Knorpelstanzvorrichtung weist eine Außenstanzvorrichtung 10, eine Öffnungsstanzvorrichtung 20 und eine Ausstoßvorrichtung 30 auf.

Die Außenstanzvorrichtung 10 weist einen Stanzfortsatz 12 und einen innen hohl ausgebildeten Außenstanzgriff 14 auf. Die Öffnungsstanzvorrichtung 20 weist einen Öffnungsstanzvorrichtungsgriff 22, eine daran angebrachte stabförmige Verlängerung 24 und einen an deren Spitze angebrachten Öffnungsstanzstab 26, mit dem die Öffnung im Knorpelplättchen ausgestanzt wird, auf. In der hier dargestellten Ausführungsform müssen der Öffnungsstanzvorrichtungsgriff 22 und die stabförmige Verlängerung 24 innen hohl ausgebildet sein, damit eine Ausstoßstange 32, die an einem Ausstoßgriff 34 der Ausstoßvorrichtung 30 befestigt ist, in ihnen beim Zusammenbau der Knorpelstanzvorrichtung gleitend aufgenommen werden kann. Zwischen der Außenstanzvorrichtung 10 und der Öffnungsstanzvorrichtung 20 befindet sich noch eine Schraubenfeder 40.

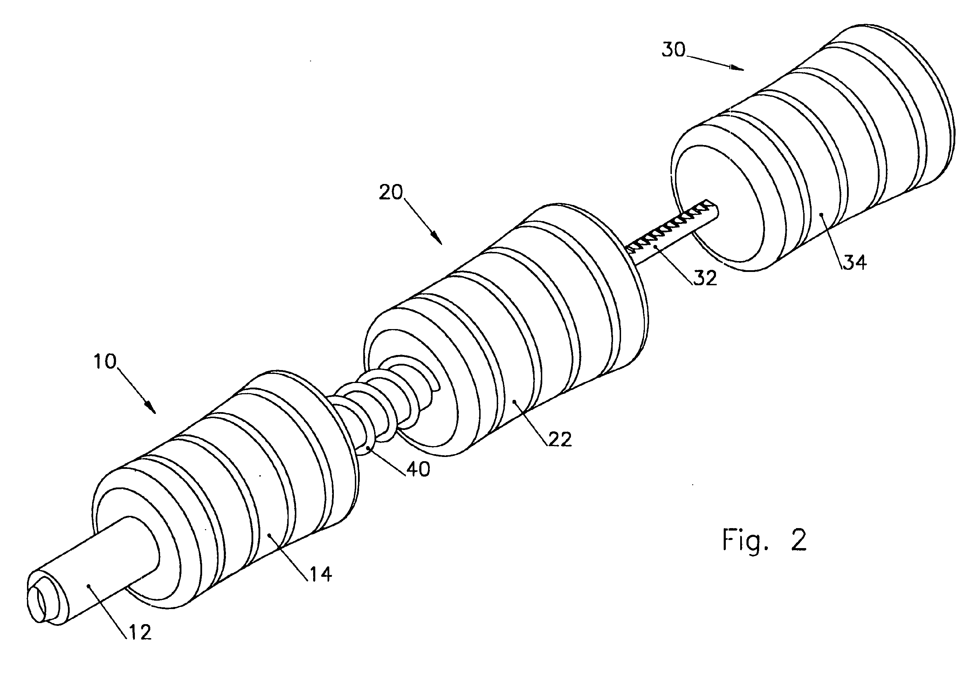

Fig. 2 zeigt den zusammengebauten Zustand der in Fig. 1 dargestellten Knorpelstanzvorrichtung. Man kann hier gut erkennen, wie die Außenstanzvorrichtung 10, die Öffnungsstanzvorrichtung 20 und die Ausstoßvorrichtung 30 in einer Linie angeordnet sind und ineinander eingreifen.

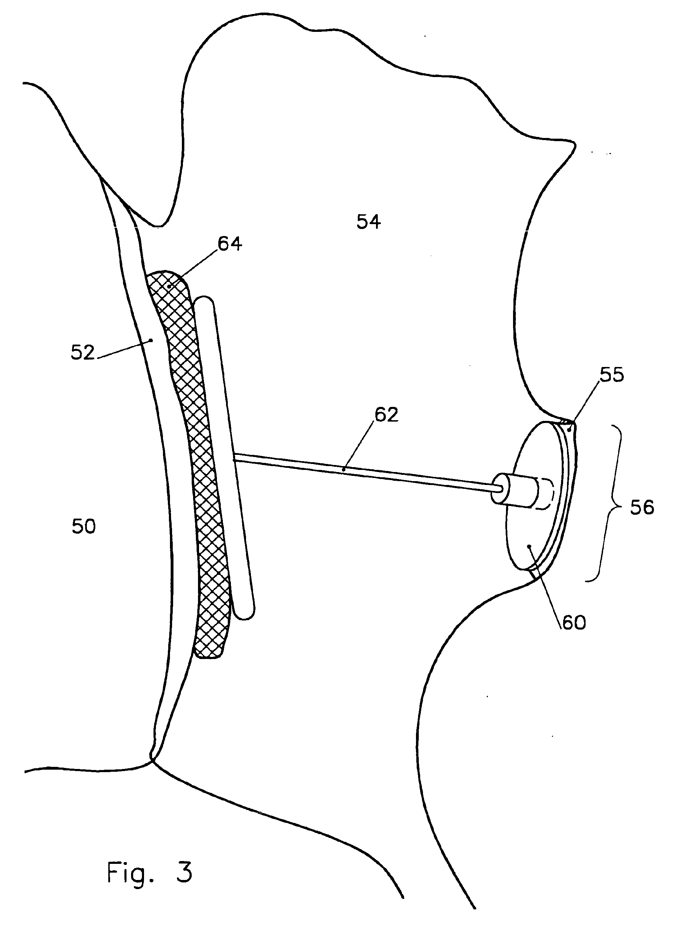

Fig. 3 zeigt einen schematischen Querschnitt durch das Mittelohr eines Menschen. Man erkennt auf der linken Seite den äußeren Gehörgang 50, dann das Trommelfell 52, das den Gehörgang 50 vom Mittelohr 54 trennt. Das Mittelohr ist auf seiner hier in der Zeichnung rechten Seite durch die Steigbügelfußplatte 55 im Bereich des ovalen Fensters 56 vom Innenohr getrennt. Im ovalen Fenster 56 ist das mit der erfindungsgemäßen Knorpelstanzvorrichtung ausgestanzte Knorpelplättchen 60 angeordnet. Es dient als Basisplatte für die Befestigung der Gehörknöchelchenprothese 62. Diese Gehörknöchelchenprothese 62 ist zwischen dem Trommelfell 52 und der Steigbügelfußplatte 55 im ovalen Fenster 56 angeordnet und dient statt der Gehörknöchelchen zur Übertragung des Schalls vom Trommelfell 52 zum Innenohr. Zwischen der Gehörknöchelchenprothese 62 und dem Trommelfell 52 ist hier noch eine Knorpelunterfütterung 64 zum Schutz des Trommelfells 52 angeordnet.

### Bezugszeichenliste :

- 10: Außenstanzvorrichtung
- 12: Stanzfortsatz
- 14: Außenstanzgriff

- 20: Öffnungsstanzvorrichtung
- 22: Öffnungsstanzvorrichtungsgriff
- 24: stabförmige Verlängerung
- 26: Öffnungsstanzstab

- 30: Ausstoßvorrichtung
- 32: Ausstoßstange
- 34: Ausstoßgriff

- 40: Feder

- 50: äußerer Gehörgang
- 52: Trommelfell
- 54: Mittelohr
- 55: Steigbügelfußplatte
- 56: ovales Fenster

- 60: Knorpelplättchen
- 62: Gehörknöchelchenprothese
- 64: Knorpelunterfütterung

## Patentansprüche

1. Knorpelstanzvorrichtung zur Herstellung eines mit einer Öffnung versehenen Knorpelplättchens wobei sie eine Außenstanzvorrichtung (10) für das Ausstanzen des Knorpelplättchens, eine Öffnungsstanzvorrichtung (20) für das Stanzen der Öffnung und eine Ausstoßvorrichtung (30) aufweist.

2. Knorpelstanzvorrichtung nach Anspruch 1, **dadurch gekennzeichnet, dass** die Außenstanzvorrichtung (10), die Öffnungsstanzvorrichtung (20) und die Ausstoßvorrichtung (30) in einer Linie hintereinander angeordnet sind.

3. Knorpelstanzvorrichtung nach Anspruch 2, **dadurch gekennzeichnet, dass** die Öffnungsstanzvorrichtung (20) einen innen hohl ausgebildeten Öffnungsstanzvorrichtungsgriff (22) und eine stabförmige Verlängerung (24), an deren Spitze ein Öffnungsstanzstab (26) zum Stanzen der Öffnung angeordnet ist, aufweist, wobei die stabförmige Verlängerung (24) in die innen hohl ausgebildete Außenstanzvorrichtung (10) gleitend eingeführt ist.

4. Knorpelstanzvorrichtung nach Anspruch 3, **dadurch gekennzeichnet, dass** die Ausstoßvorrichtung (30) einen Ausstoßgriff (34) und eine daran befestigte Ausstoßstange (32) aufweist, und die stabförmige Verlängerung (24) der Öffnungsstanzvorrichtung (20) innen hohl ausgebildet ist, wobei die Ausstoßstange (32) in die innen hohl ausgebildete stabförmige Verlängerung (24) der Öffnungsstanzvorrichtung (20) gleitend eingeführt ist.

5. Knorpelstanzvorrichtung nach einem der vorherigen Ansprüche, **dadurch gekennzeichnet, dass** die Außenstanzvorrichtung (10) einen innen hohl ausgebildeten Außenstanzgriff (14) und einen daran angeordneten innen hohl ausgebildeten Stanzfortsatz (12) aufweist.

6. Knorpelstanzvorrichtung nach einem der vorherigen Ansprüche, **dadurch gekennzeichnet, dass** eine Feder (40) zwischen der Außenstanzvorrichtung (10) und der Öffnungsstanzvorrichtung (20) angeordnet ist.

7. Knorpelstanzvorrichtung nach Anspruch 6, **dadurch gekennzeichnet, dass** die Feder (40) in Form einer Schraubenfeder ausgebildet ist.

## Claims

1. Cartilage punching device for manufacturing a cartilage plate provided with an opening, wherein it exhibits an external punching device (10) for punching out the cartilage plate, an opening punching device (20) for punching the opening, and an ejector device (30).

2. Cartilage punching device according to Claim 1, **characterised in that** the external punching device (10), the opening punching device (20) and the ejector device (30) are arranged one behind the other in a line.

3. Cartilage punching device according to Claim 2, **characterised in that** the opening punching device (20) exhibits an opening punching device handle (22) that is hollow on the inside and a rod-shaped extension (24) at whose tip an opening punching rod (26) is arranged for punching the opening, wherein the rod-shaped extension (24) is slid into the outer punching device (10) that is hollow on the inside.

4. Cartilage punching device according to Claim 3, **characterised in that** the ejector device (30) exhibits an ejector handle (34) and an ejector rod (32) attached to it, wherein the rod-shaped extension (24) of the opening punching device (20) is hollow on the inside, and wherein the ejector rod (32) is slid into the rod-shaped extension (24) of the opening punching device (20), which extension is hollow on the inside.

5. Cartilage punching device according to any one of the preceding claims, **characterised in that** the external punching device (10) exhibits an external punching handle (14) that is hollow on the inside and a punching projection (12) that is arranged on it and is also hollow on the inside.

6. Cartilage punching device according to any one of the preceding claims, **characterised in that** a spring (40) is arranged between the external punching device (10) and the opening punching device (20).

7. Cartilage punching device according to Claim 6, **characterised in that** the spring (40) is designed in the form of a helical spring.

## Revendications

1. Dispositif de découpage de cartilage pour réaliser une plaquette de cartilage pourvue d'une ouverture, lequel comporte un dispositif de découpage extérieur (10) pour le découpage de la plaquette de cartilage, un dispositif de découpage d'ouverture (20) pour le découpage de l'ouverture et un dispositif d'éjection (30).

2. Dispositif de découpage de cartilage selon la revendication 1, **caractérisé en ce que** le dispositif de découpage extérieur (10), le dispositif de découpage d'ouverture (20) et le dispositif d'éjection (30) sont disposés en ligne l'un derrière l'autre.

3. Dispositif de découpage de cartilage selon la revendication 2, **caractérisé en ce que** le dispositif de découpage d'ouverture (20) comporte une poignée (22) réalisée creuse intérieurement et un prolongement (24) en forme de barre à la pointe duquel est disposée une barre de découpage d'ouverture (26) pour découper l'ouverture, le prolongement (24) en forme de barre étant introduit avec glissement dans le dispositif de découpage extérieur (10) réalisé creux à l'intérieur.

4. Dispositif de découpage de cartilage selon la revendication 3, **caractérisé en ce que** le dispositif d'éjection (30) comporte une poignée d'éjection (34) et une tige d'éjection (32) fixée à celle-ci, et le prolongement (24) en forme de barre du dispositif de découpage d'ouverture (30) est réalisé creux à l'intérieur, la tige d'éjection (32) étant introduite avec glissement dans le prolongement (24) en forme de barre, réalisé creux à l'intérieur, du dispositif de découpage d'ouverture (20).

5. Dispositif de découpage de cartilage selon l'une des revendications précédentes, **caractérisé en ce que** le dispositif de découpage extérieur (10) comporte une poignée de découpage extérieur (14) réalisée creuse à l'intérieur et un prolongement de découpage (12) disposé sur celle-ci et réalisé creux à l'intérieur.

6. Dispositif de découpage de cartilage selon l'une des revendications précédentes, **caractérisé en ce qu'**un ressort (40) est disposé entre le dispositif de découpage extérieur (10) et le dispositif de découpage d'ouverture (20).

7. Dispositif de découpage de cartilage selon la revendication 6, **caractérisé en ce que** le ressort (40) est réalisé sous la forme d'un ressort hélicoïdal.
